(19) Europäisches Patentamt
European Patent Office
Office européen des brevets

(11) **EP 4 059 493 A1**

(12) **EUROPEAN PATENT APPLICATION**

(43) Date of publication:
**21.09.2022  Bulletin 2022/38**

(21) Application number: **21162765.8**

(22) Date of filing: **16.03.2021**

(51) International Patent Classification (IPC):
**A61K 9/20** *(2006.01)*     **A61K 31/555** *(2006.01)*
**A61P 11/00** *(2006.01)*     **A61P 31/14** *(2006.01)*

(52) Cooperative Patent Classification (CPC):
**A61K 9/2054; A61K 31/555; A61P 31/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **THE SECRETARY, DEPARTMENT OF ATOMIC ENERGY**
**Mumbai 400 001 (IN)**

(72) Inventors:
• **Sharma, Deepak**
  **400085 Mumbai (IN)**
• **Sandur, Santosh Kumar**
  **400085 Mumbai (IN)**
• **Gota, Vikram Prakash**
  **410210 Navi Mumbai (IN)**

• **Patwardhan, Raghavendra Shridhar**
  **400085 Mumbai (IN)**
• **Singh, Babita**
  **400085 Mumbai (IN)**
• **Checker, Rahul**
  **400085 Mumbai (IN)**
• **Maurya, Dharmendra Kumar**
  **400085 Mumbai (IN)**
• **Joshi, Mahendra**
  **560099 Bengaluru (IN)**
• **Giri, Amarjit**
  **560099 Bengaluru (IN)**
• **Madki, Shivkumar**
  **560099 Bengaluru (IN)**
• **Gupta, Sudeep**
  **410210 Navi Mumbai (IN)**

(74) Representative: **Petraz, Gilberto Luigi et al**
**GLP S.r.l.**
**Viale Europa Unita, 171**
**33100 Udine (IT)**

(54) **A CHLOROPHYLLIN CONTAINING PHARMACEUTICAL COMPOSITION FOR PREVENTION OF PATHOGENESIS OF CORONAVIRUS DISEASE**

(57)     Pharmaceutical composition comprises of Chlorophyllin or salts thereof and its utilities including method of treatment, enabling the absorption of Copper-isochlorin e4 into human blood that results in an increase in lymphocyte count and decrease in the abundance of hematopoietic stem and progenitor cells (HPSCs) in human blood. The invention is directed to the treatment of conditions including corona-virus infection, immunosuppression, leucopenia and lymphopenia. The composition disclosed in the invention is thus useful for the treatment of corona virus disease caused by SARS-CoV-2 infection by decreasing viral infection, reducing cytokine storm and pro-inflammatory chemokines, reducing epithelial-cell oxidative-stress in the lungs and increasing the production of leukocytes.

FIGURE 1

EP 4 059 493 A1

**Description**

**FIELD OF THE INVENTION**

This invention relates to a pharmaceutical composition for prevention of pathogenesis of corona virus infection and disease in human. The said pharmaceutical composition comprises a Chlorophyllin or salts thereof enabling the absorption of copper-isochlorin e4 into human blood, that results in an increase in lymphocyte count and decrease in the abundance of hematopoietic stem and progenitor cells (HSPCs) in human blood. The invention is directed to treatment of conditions including corona-virus infection, immune suppression, leucopenia and lymphopenia. The composition disclosed in the invention is thus useful for the treatment of corona virus disease including SARS-CoV-2 infection by decreasing viral infection, reducing cytokine storm and inflammatory chemokines, reducing epithelial-cell oxidative-stress in lungs and increasing the production of leukocytes.

**BACKGROUND ART**

Chlorophyllin is a water-soluble derivative of green plant pigment chlorophyll and it is available as an over the counter drug under brand name Derifil which falls under the class of GRAS (grossly recognized as safe) drugs. Chlorophyllin was shown to act as an antioxidant and thereby prevent radiation induced damage to biomolecules like DNA in cell free systems. Chlorophyllin was also shown to enhance the immune response in mice. Various studies have shown the antioxidant, radio protective, immune-modulatory and anti-apoptotic effects of chlorophyllin in mice. Chlorophyllin has also been shown to increase the abundance of hematopoietic stem cells in the bone marrow and protect mice against radiation induced bone marrow aplasia, lung epithelial cellular atypia, oxidative stress and mortality in mice (1,2). Antiviral activity of Chlorophyllin has been reported against hepatitis C virus, polio virus and bovine herpes virus (3-7). However, there is no information on systemic availability and pharmacodynamics of Chlorophyllin in human (8).

**Drawbacks connected with hitherto known Processes/devices:**

Corona virus disease (COVID-19) is an infectious disease caused by the recently discovered SARS-CoV-2 virus. In human the SARS-CoV-2 virus infects lower part of lungs and causes respiratory illness with flu like symptoms such as cough, fever, and in more severe cases, difficulty in breathing, acute respiratory distress syndrome (ARDS) and death. SARS-CoV-2 infection causes damage to lung epithelial cells, oxidative stress, increase in inflammatory cytokines and chemokines, decrease in leukocyte count and death of lymphocytes in human (9). The viral trimeric spike protein binds to the human receptor angiotensin-converting enzyme 2 (ACE2). ACE 2 is a type I membrane protein expressed in lungs, heart, kidneys, and intestine and it is involved in the maturation of angiotensin, a peptide hormone that controls vasoconstriction and blood pressure. The receptor binding domain (RBD) of the S1 subunit of surface spike glycoprotein (S protein) of SARS-CoV-2 virus mediates host cell receptor binding to the peptidase domain (PD) of angiotensin-converting enzyme 2 followed by internalization into the cells via S2 dependent membrane fusion (10). Presently more than 120 potential COVID-19 treatments are being tested, which include repurposing of drugs used for HIV and malaria, experimental compounds that work against an array of viruses in animal experiments, and antibody-rich plasma from people who have recovered from COVID-19.

However, there is no medicine to prevent or treat corona virus disease and hence there is an urgent need to develop new medicaments for COVID-19. There is a highest requirement in the art for a medicine or pharmaceutical composition or a dosage form comprising medicine for the treatment for COVID-19 infection.

It is not obvious that properties of chlorophyllin should protect against SARS-CoV-2 infection induced disease (COVID-19) and their morbidity and mortality. There are no reports on the effect of chlorophyllin protecting against Coronavirus (COVID-19) infectious diseases induced mortality. Further, there is no information on systemic availability and pharmacodynamics of chlorophyllin in human. The existing formulations of chlorophyllin which are available in the market or referred in the literature have never been reported to increase the serum concentration of copper isochlorin e4 to 5-35 micro molar concentration in human blood. The prior art suggests that existing chlorophyllin tablets can achieve a steady state concentration of copper chlorin e4 ethyl ester (molecular weight 641.4) to 1-2 microgram / ml (about 1.5 to 3 micro molar) concentration in human blood [Chem. Res. Toxicol. 2000, 13, 9, 900-906]. It is not obvious from any prior art that copper-chlorin e4 ethyl ester has any effect on SARS-CoV-2 virus. There are no reports showing increase in concentration of sodium copper-isochlorin-e4 to more than 5 micro-molar using the Chlorophyllin tablets available in the market which is necessary for inhibiting multiplication of SARS-CoV-2 Virus.

**OBJECT OF THE INVENTION**

One of the basic objects of the present invention is to provide a pharmaceutical composition to improve bioavailability of chlorophyllin by oral route.

Another basic object of the present invention is to provide a pharmaceutical composition for treatment of COVID-19 infections and diseases with such compositions.

Another object is to provide a pharmaceutical composition comprising Chlorophyllin or pharmaceutically acceptable salts thereof in the form of oral tablets with defined pharmacodynamics and immune modulatory properties

A further object of the present invention is to provide a pharmaceutical composition comprising Chlorophyllin which achieves the therapeutic plasma concentration of the active pharmacological ingredient (API) copper isochlorin e4 in human blood which inhibit SARS-CoV-2 infection.

Yet another object of the present invention is to use the said pharmaceutical composition for inducing 10 to 25% increase in blood neutrophil counts, decrease in pro-inflammatory chemokines such as (1) chemokine ligand 2 or Monocyte Chemoattractant Protein-1 (MCP-1); (2) Chemokine (C-C motif) ligand-3 or Macrophage Inflammatory Protein MIP-1$\alpha$ and (3) Chemokine (C-C motif) ligand 4 or Macrophage Inflammatory Protein MIP-1$\beta$) which are associated with pathogenesis of COVID-19 in human.

Another object of the present invention is to use the said composition comprising Chlorophyllin for reducing the abundance of CD34+ hematopoietic stem and progenitor cells(HSPCs) in human blood.

A still further object of the present invention is to provide a pharmaceutical composition comprising chlorophyllin which induces up to 30% increase in human blood lymphocyte counts.

The present invention relates to solid oral pharmaceutical composition comprising chlorophyllin and one or more pharmaceutically acceptable excipients.

Another object of the present invention is to provide a method of treatment of SARS-CoV-2 infection in human subjects by administering orally a specific dose of the said pharmaceutical composition.

## SUMMARY OF INVENTION

The basic aspect of the present invention is thus to provide a chlorophyllin containing pharmaceutical composition for prevention of pathogenesis of coronavirus disease in humans comprising: Chlorophyllin or salts thereof in amounts between 20 to 80% by wt; and a pharmaceutically acceptable adjuvant.

Another aspect of the present invention provides a chlorophyllin containing pharmaceutical composition comprising water-soluble derivative of green plant pigment chlorophyll wherein the composition is either a solid oral dosage form or liquid oral dosage form suitable for oral administration.

Yet another aspect of the present invention provides a chlorophyllin containing pharmaceutical composition wherein the composition is a tablet dosage form, preferably a coated tablet.

A still further aspect of the present invention provides a chlorophyllin containing pharmaceutical composition, which achieves serum concentration of an active pharmacological ingredient copper isochlorin e4 up to a concentration range of 5 to 35 micro-molar in human blood to inhibit SARS-CoV-2 infection.

In another aspect, the present invention provides a chlorophyllin containing pharmaceutical composition which induces 10 to 25% increase in blood neutrophil counts, decrease in pro-inflammatory chemokines (1) chemokine ligand 2 or Monocyte Chemoattractant Protein-1 (MCP-1); (2) Chemokine (C-C motif) ligand-3 or Macrophage Inflammatory Protein (MIP-1-alpha) and (3) Chemokine (C-C motif) ligand 4 or Macrophage Inflammatory Protein (MIP-1-beta) which are associated with pathogenesis of COVID-19 in human.

A still further aspect of the present invention provides the chlorophyllin containing pharmaceutical composition according to claim 1 favoring selectively anyone or more of:

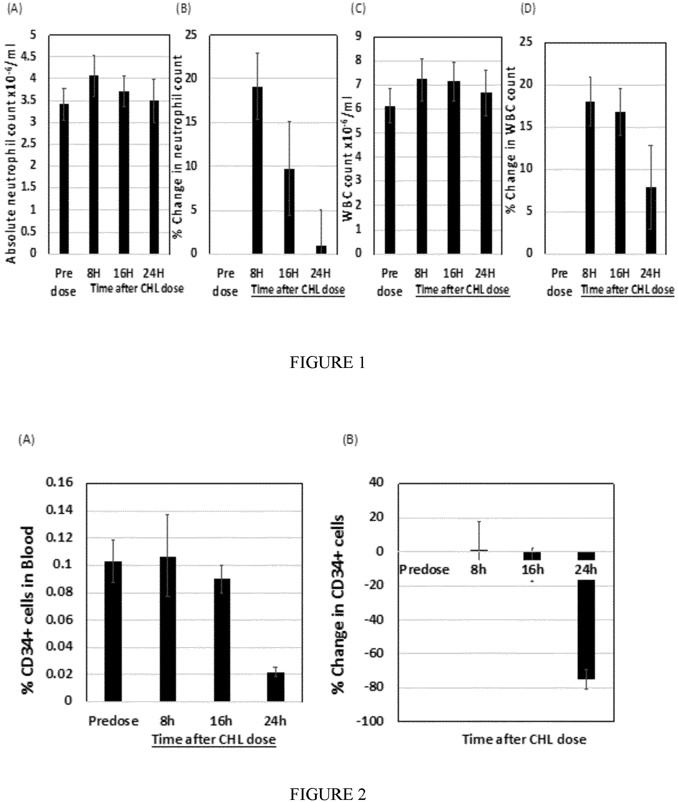

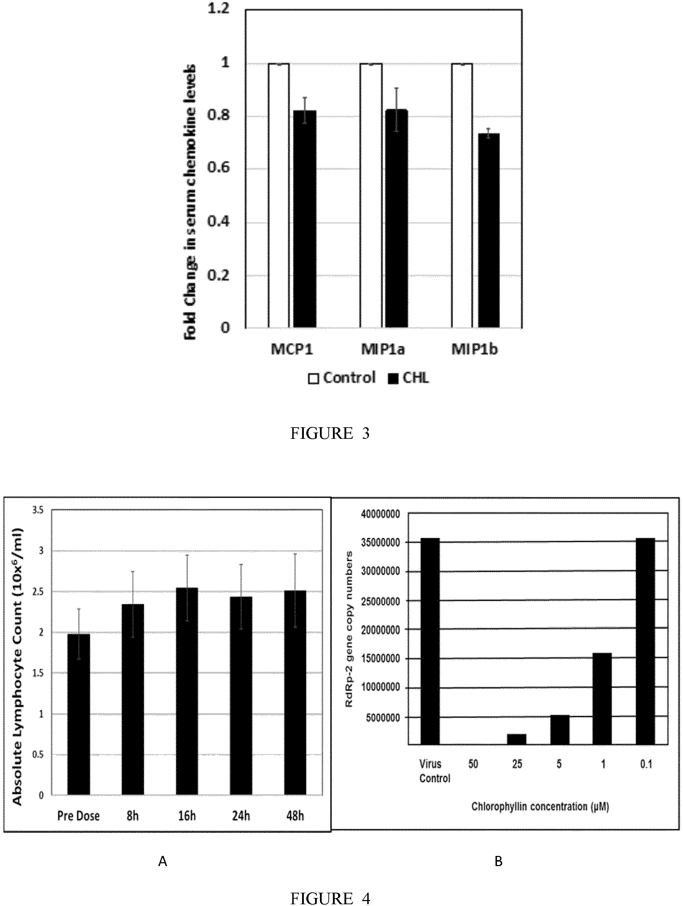

i) increase in absolute neutrophil count (ANC) between 12.4-32.5% relative to pre-dose value at 8-16 hr after first dose of Chlorophyllin(750mg) (Fig. 1A, 1B)];
ii) increase in WBC count between 12.8-26.5% relative to pre-dose value at 8-16hr after first dose of Chlorophyllin (Fig 1C ,1D);
iii) decreasing the abundance of CD34+ hematopoietic stem and progenitor cells in the blood between 50-85% as compared to pre-dose value (Fig 2) in human subjects;
iv) decreasing the concentration of pro-inflammatory chemokines MCP-1, MIP-1 alpha and MIP-1 beta between 13-37% as compared to pre-dose value 24 hr after first dose of Chlorophyllin (Fig 3);
v) relative decrease in MIP-1 alpha in human blood between 12-21% as compared to pre-dose value 72 hr after first dose of Chlorophyllin;
vi) relative decrease in MIP-1 beta in human blood was between 11-55% as compared to pre-dose value 24 hr after first dose of Chlorophyllin.
vii) inducing up to 30% increase in human blood lymphocyte counts.

In a further aspect the present invention provides a method of treatment of disease condition including pathogenesis of coronavirus disease in humans comprising administering preferably via oral administration of pharmaceutically effective amount of Chlorophyllin or salts thereof at a dose of 500 to 1500 mg, preferably 750 mg for 3 to 14 days to human adult weighing 50-100 Kg to thereby induce an increase in the serum concentration of an active pharmacological ingredient (API) copper isochlorin e4 up to a concentration range of 5 to 35 micro M in human blood to inhibit SARS-CoV-2 infection.

Another aspect of the present invention provides the method comprising of administration of one tablet containing at least 500 mg preferably about 750 mg Chlorophyllin every day in the morning for 3 to 14 consecutive days with a dose regimen wherein the tablet must be taken 10 to 12 hr after overnight fasting along with water and food should be consumed 2 hr after administration of the Chlorophyllin tablet. A further aspect of the present invention provides the method comprising administering said dosage of Chlorophyllin further provides for reducing the abundance of CD34+ hematopoietic stem and progenitor cells (HSPCs) in human blood.

In another aspect, the present invention provides the method comprising administering said dosage of chlorophyllin further for inducing up to 30% increase in human blood lymphocyte counts.

In a further aspect, the present invention provides the method comprising administering said dosage further for inducing 10 to 25% increase in blood neutrophil counts, decrease in pro-inflammatory chemokines (1) chemokine ligand 2 or Monocyte Chemoattractant Protein-1 (MCP-1); (2) Chemokine (C-C motif) ligand-3 or Macrophage Inflammatory Protein MIP-1-alpha and (3) Chemokine (C-C motif) ligand 4 or Macrophage Inflammatory Protein MIP-1-beta) which are associated with pathogenesis of COVID-19 in human.

In yet another aspect, the present invention provides the method comprising administering said dosage thereby favoring selectively anyone or more of :

    i) increase in absolute neutrophil count (ANC) between 12.4-32.5% relative to pre-dose value at 8-16 hr after first dose of Chlorophyllin (750 mg);
    ii) increase in WBC count between 12.8-26.5% relative to pre-dose value at 8-16 hr after first dose of Chlorophyllin (Fig 1C, 1D);
    iii) decreasing the abundance of CD34+ hematopoietic stem and progenitor cells in the blood between 50-85% as compared to pre-dose value (Fig 2) in human subjects;
    iv) decreasing the concentration of pro-inflammatory chemokines MCP-1, MIP-1-alpha and MIP-1-beta between 13-37% as compared to pre-dose value 24 hr after first dose of Chlorophyllin (Fig 3);
    v) relative decrease in MIP-1-alpha in human blood between 12-21% as compared to pre-dose value 72 hr after first dose of Chlorophyllin;
    vi) relative decrease in MIP-1-beta in human blood was between 11-55% as compared to pre-dose value 24 hr after first dose of Chlorophyllin;

Still another aspect of the present invention provides Chlorophyllin or salts thereof in amounts between 20 to 80% by wt. and pharmaceutically acceptable adjuvant in dosage level of 500 to 1500 mg, for use in treatment of pathogenesis of coronavirus disease in humans by inducing an increase in the serum concentration of an active pharmacological ingredient (API) Copper isochlorin e4 up to a concentration range of 5 to 35 micro M in human blood to inhibit SARS-CoV-2 infection.

Another aspect of the present invention provides the Chlorophyllin or salts thereof as claimed in claim 13 for use either as a solid oral dosage form or liquid oral dosage form suitable for oral administration.

Yet another aspect of the present invention provides the chlorophyllin or salt thereof as claimed in claim 13 for inducing 10 to 25% increase in blood neutrophil counts, decrease in pro-inflammatory chemokines (1) chemokine ligand 2 or Monocyte Chemoattractant Protein-1 (MCP-1); (2) Chemokine (C-C motif) ligand-3 or Macrophage Inflammatory Protein (MIP-1-alpha) and (3) Chemokine (C-C motif) ligand 4 or Macrophage Inflammatory Protein (MIP-1-beta) which are associated with pathogenesis of COVID-19 in human.

A further aspect of the present invention provides the chlorophyllin or salt thereof for selectively anyone or more of:

    i) increase in absolute neutrophil count (ANC) between 12.4-32.5% relative to pre-dose value at 8-16 hr after first dose of Chlorophyllin(750mg), and 4hr before second dose(Fig 1A, 1B);
    ii) increase in WBC count between 12.8-26.5% relative to pre-dose value at 8-16 hr after first dose of Chlorophyllin (Fig 1C, 1D);
    iii) decreasing the abundance of CD34+ hematopoietic stem and progenitor cells in the blood between 50-85% as compared to pre-dose value (Fig 2) in human subjects;
    iv) decreasing the concentration of pro-inflammatory chemokines MCP-1, MIP-1-alpha and MIP-1-beta between 13-37% as compared to pre-dose value 24 hr after first dose of Chlorophyllin (Fig 3);
    v) relative decrease in MIP-1-alpha in human blood between 12-21% as compared to pre-dose value 72 hr after first dose of Chlorophyllin;
vi) relative decrease in MIP-1-beta in human blood was between 11-55% as compared to pre-dose value 24 hr after first dose of Chlorophyllin.

Yet another aspect of the present invention provides Chlorophyllin or salt thereof for use in manufacture of medicament in amounts between 20 to 80% by wt. and pharmaceutically acceptable adjuvant in dosage level of 500 to 1500 mg, for treatment of pathogenesis of coronavirus disease in humans by inducing an increase in the serum concentration of an active pharmacological ingredient (API) Copper isochlorin e4 up to a concentration range of 5 to 35 micro M in human blood to inhibit SARS-CoV-2 infection.

In another aspect the present invention provides the chlorophyllin containing pharmaceutical composition which increases bioavailability of the Chlorophyllin in human subjects.

## BRIEF DESCRIPTION OF FIGURES

**Fig.1** represents effect of Chlorophyllin formulation on neutrophil and WBC abundance in healthy human subjects (N=5; Average $\pm$SEM).

**Fig.2** represents effect of Chlorophyllin formulation on hematopoietic stem and progenitor cell abundance in healthy human subjects (N=5; Average $\pm$SEM).

**Fig.3** represents effect of Chlorophyllin formulation on serum concentration of MCP1 , MIP1$\alpha$ and MIP 1$\beta$ in healthy human subjects (N=5;Average$\pm$SEM).

**Fig.4** represents effect of Chlorophyllin formulation on absolute lymphocyte count in healthy human subjects (N=5; Average$\pm$SEM) and effect of Chlorophyllin on SARS-CoV-2 virus multiplication in VeroCCL-81 cells in vitro. 4(A) illustrates CHL administration increases absolute lymphocyte counts in human blood. 4(B) illustrates CHL decreases SARS-CoV-2 virus replication in Vero CCL-81 cells in a dose dependent manner.

**Fig.5** illustrates Mean serum concentration of Copper isochlorin e4 under fasting conditions. Administration of oral tablets of CHL increases the concentration of copper isochlorin e4 in human blood.

**Fig. 6** illustrates pharmacokinetics of Chlorophyllin in human healthy subjects with food effect; represents Serum concentration of Copper isochlorin e4 in human serum under fed conditions.

## DETAIL DESCRIPTION OF INVENTION

The present advancement is thus directed to a pharmaceutical composition comprising a synergistic combination of Chlorophyllin or salts therof and Carrier /Binding material in select concentration and method of treatment involving the said chlorophyllin containing therapeutic preparation in oral tablet form which enables increasing the abundance of neutrophils and decreasing the abundance of hematopoietic stem cells in the blood and decreasing the levels of pro-inflammatory chemokines MCP-1, MIP-1$\alpha$ and MIP-1$\beta$ in human serum. These parameters can alleviate virus induced leucopenia and inflammatory cytokine syndrome in COVID-19 patients.

This formulation achieves serum concentration of an active pharmacological ingredient (API) Copper isochlorin e4 in human blood to a specific concentration which inhibits SARS-CoV-2 infection. This formulation also induces up to 24% increase in blood neutrophil production, decrease in pro-inflammatory chemokines (1) chemokine ligand 2 or Monocyte Chemoattractant Protein-1 (MCP-1); (2) Chemokine (C-C motif) ligand-3 or Macrophage Inflammatory Protein MIP-1-alpha and (3) Chemokine (C-C motif) ligand-4 or Macrophage Inflammatory Protein MIP-1-beta) which are associated with pathogenesis of COVID-19 in healthy human subjects. Further, this formulation also reduces the abundance of CD34+ hematopoietic stem and progenitor cells (HPSCs) in human blood. Chlorophyllin treatment induces up to 30% increase in human blood lymphocyte counts. The faster recovery from lymphocytopenia may also underline the therapeutic effects of Chlorophyllin tablets in COVID-19 patients. One of the basic aspect of the present invention provides the use of Chlorophyllin or pharmaceutically acceptable salts thereof as a medicament for prevention, mitigation and treatment of corona virus diseases in human comprising of administration of one tablet containing 750 mg Chlorophyllin every day in the morning for three to fourteen consecutive days with a dose regimen wherein the tablet must be taken 10 to 12 hr after overnight fasting along with water and food should be consumed 2 hr after administration of the Chlorophyllin tablet.

Enhancement of Sodium Copper isochlorin e4 in human serum to the specific level has not been reported using the existing Chlorophyllin tablets. Further, there is no prior art on use of 500-1500 mg dose of chlorophyllin per day in human subjects. This dose range was optimized by conducting a Phase I clinical trial study in human subjects in India and it is thus a technical advance in the related art. The existing CHL tablets of 100 mg size are known to increase the serum concentration of Sodium Copper chlorin e4 ethyl ester up to 3 microM which does not have inhibitory effect on SARS-CoV-2 multiplication. Surprisingly and unexpectedly it has been found by way of the present invention that the oral composition of the present invention increased the serum concentration of sodium Copper isochlorin e4 to the specific level but not of copper-chlorin e4 ethyl ester in human serum in Phase I clinical trial study in human subjects in India.

Based on topological polar surface area (170 Å2) of copper chlorophyllin, (https://pubchem.ncbi.nlm.nih.gov/compound/CopperchlorophyllinA#section= Computed-Properties) both Copper isochlorin e4 and Copper-chlorin e4 ethyl ester are supposed to have very poor bioavailability by oral route. It was observed that the tablets used in the present invention, are responsible for selective increase in sodium copper isochlorin e4 only which is essential for viral inhibition.

There is no prior art on use of chlorophyllin tablets of 500 to 1500 mg per day for increasing the serum concentration of Copper isochlorin e4 and medicament of COVID-19 disease. The prior art is mainly available on use of 50 to 300 mg dose of chlorophyllin per day for prevention of body odour in geriatric patients and also as a cancer chemo-preventive drug which can have absolutely no teachings towards the increase in serum concentration of Copper isochlorin e4. Hence, the present finding is a clear technical advance involving surprising and unexpected results due to (1) bigger size of tablets (500-750 mg) and higher dosage (500-1500 mg per day), (2) difference in pharmacokinetics of the tablets evinced from specific increase in serum concentration of Copper isochlorin e4. These findings are different and unexpected from the tablets available in prior art where the size is smaller (50-100 mg), recommended dose range is also lower (up to 300 mg per day) and the pharmacokinetics show increase in Copper chlorin e4 ethyl ester in human blood.

In another aspect the present invention provides the method for increasing the abundance of lymphocytes and decreasing the abundance of hematopoietic stem cells in the blood and decreasing the levels of pro-inflammatory chemokines MCP-1, MIP-1-alpha and MIP-1-beta in human serum.

The present invention also provides the method for increasing the serum concentration of copper isochlorin e4 in human blood up to a concentration range of 5 to 35 micro molar by administering specific dose or chlorophyllin composition which suppresses SARS-CoV-2 multiplication. The oral pharmaceutical composition comprises, chlorophyllin or a pharmaceutically acceptable salt thereof and one or more pharmaceutically acceptable excipient or carriers containing chlorophyllin or a pharmaceutically acceptable salt thereof in an amount of 10mg to 1500 mg.

Smaller tablets of chlorophyllin (for example 10mg) using the binders mentioned in the present invention can also be used for medicament of COVID-19 in the prescribed dose (10mg x 50 tablet = 500 mg per day dose or 10 mg x 75 tablets per day for 750 mg dose).

The said pharmaceutical composition of oral tablets of the present invention allows absorption of copper isochlorin e4 in to human blood thereby increasing its serum concentration which is distinct from other tablets available in the market which are known to increase serum concentration of copper-chlorin e4 ethyl ester in human blood. The oral pharmaceutical composition comprises solid and liquid oral dosage forms. The present invention provides a unique pharmaceutical composition for prevention and treatment of COVID-19 disease by prevention of viral entry into human cells, reduction of lung epithelial cell damage and treatment of SARS-CoV-2 induced lymphopenia/ neutropenia and inflammation. At present, there are no drugs specifically approved for treatment of COVID-19 disease. The present invention provides medicinal use of a defined dose of Chlorophyllin composition in the form of oral tablets for prevention, and treatment of corona virus diseases including COVID-19 disease in humans.

The advancement under the present invention demonstrates for the first time the use of chlorophyllin containing pharmaceutical composition which achieves the serum concentration of copper isochlorin e4 in human blood up to a concentration range of 5-35µM which can inhibit SARS-CoV-2 infection in human. The evidence of efficacy of chlorophyllin is provided in terms of inhibition of SARS-CoV-2 virus multiplication, which causes COVID-19 disease. The prior art suggests that existing chlorophyllin tablets can achieve a steady state concentration of copper chlorin e4 ethyl ester to about 3 micromolar concentration in human blood. [Chem. Res. Toxicol. 2000, 13, 9, 900-906] which is not effective in inhibiting SARS-CoV-2 infection in human.

Examples of pharmaceutical composition comprising chlorophyllin include tablets, granules, fine granules, powders, capsules, chewable tablets, effervescent tablets, dispersible tablets, mouth dissolving tablets, pellets, syrups, solutions, suspensions . The oral solid composition of the present invention comprises a specific dose of chlorophyllin and pharmaceutical excipients suitable for oral administration. Such excipients are selected from the group consisting of binding agents, fillers, filler-binders, disintegrant, lubricants, sweeteners, flavoring and coloring agents, preferably the excipients are selected from the group consisting of binding agents, filler-binders, and lubricants.

The fillers and /or filler-binder are selected from the group consisting of different grades of starches, such as maize starch, potato starch, rice starch, wheat starch, pre-gelatinized starch, fully pre-gelatinized starch, cellulose, such as microcrystalline cellulose or silicified microcrystalline cellulose, mannitol, erythritol, lactose, such as lactose monohydrate and lactose anhydrous, calcium salts, such as calcium hydrogenphosphate dihydrate, anhydrous dibasic calcium phosphate, sorbitol, and xylitol, particularly preferred, the fillers and/or filler-binders are selected from the group consisting of pregelatinized starch, microcrystalline cellulose, lactose monohydrate, and lactose, even further preferred the filler and /or filler-binder is selected from the group consisting of microcrystalline cellulose and anhydrous dibasic calcium phosphate.

Binding agents are selected from the group consisting of polyvinylpyrrolidone (Povidone), copolymers of vinylpyrrolidone with other vinyl derivatives (Copovidone), hydroxypropylmethylcellulose, methylcellulose, hydroxy propylcellulose, powdered acacia, gelatin, guargum, carbomer such as carbopol, polymethacrylates and starch.

In an embodiment, the immediate release (second and/or third) compartment additionally comprises disintegrant.

The solid oral pharmaceutical composition of the present invention can be prepared by methods known to the person skilled in the art. Preferably, the composition comprising chlorophyllin or pharmaceutically acceptable salts thereof are formed by dry granulation, wet granulation, slugging or direct compression or by coating process. The compartments then can be processed in different orders and methods known to the person skilled in the art to form a dosage form.

The term "pharmaceutically acceptable" means that which is generally safe, non-toxic and neither biologically nor otherwise undesirable and includes that which is acceptable for human pharmaceutical use as well as veterinary use. In the blend of the present invention, suitable pharmaceutically acceptable excipients include, but are not limited to, binders, diluents, disintegrants, lubricants, fillers, carriers, and the like. The term "pharmaceutically acceptable salts" means sodium or potassium salts of copper chlorophyllin.

Binders are used to impart cohesive qualities to a solid (tablet) formulation, and thus ensure that a tablet remains intact after compaction. Suitable binder materials include, but are not limited to, microcrystallinecellulose, gelatin, sugars (including sucrose, glucose, dextrose and maltodextrin), polyethyleneglycol, waxes, natural and synthetic gums, polyvinylpyrrolidone, cellulosicpolymers (including hydroxyl propylcellulose, hydroxypropylmethylcellulose, methylcellulose, hydroxylethyl cellulose, and the like). The diluents employed in a composition of the present invention may be one or more compounds which are capable of providing compactability and good flow. A variety of materials may be used as fillers or diluents. Suitable diluents or fillers include, but are not limited to, lactose (monohydrate, spray-dried monohydrate, anhydrous and the like), sucrose, dextrose, mannitol, sorbitol, starch, cellulose (e.g. microcrystallinecellulose; Avicel®), dehydrated or anhydrous dibasic calcium phosphate, calcium carbonate, calcium sulfate, and others as known in the art.

Lubricants can be employed herein in the manufacture of certain dosage forms, and will usually be employed when producing granules and tablets. In the present invention, a lubricant is typically added just prior to slugging or compacting to form the granule, and is mixed with the formulation for a minimum period of time to obtain good dispersal. The lubricant employed in the present invention may be one or more compounds. Examples of suitable lubricants include, but are not limited to, magnesium stearate, calcium stearate, zinc stearate ,stearic acid, talc, glycerylbehenate, polyethyleneglycol, polyethyleneoxidepolymers (for example, available under the registered trademarks of Carbowax™ forpolyethylene glycol and Polyox™ for polyethylene oxide from Dow Chemical Company, Midland,Mich.), sodium lauryl sulfate ,magnesium laurylsulfate, sodiumoleate, sodium stearyl fumarate, DL-leucine, colloidal silica, and others as known in the art. Preferred lubricants are magnesium stearate and mixtures of magnesium stearate with sodiumlauryl sulfate. Lubricants may comprise from about 0.1wt% to about 8.0wt% of the granule weight.

Disintegrants are used to facilitate tablet disintegration or "breakup" after administration, and are generally starches, clays, celluloses, algins, gums or cross-linked polymers. Suitable disintegrants include, but are not limited to, cross-linked polyvinylpyrrolidone(PVP-XL), sodium starch glycolate, and croscarmellose sodium. If desired, the pharmaceutical formulation may also contain minor amounts of nontoxic auxiliary substances such as wetting or emulsifying agents, pH buffering agents and the like, for example, sodium acetate, sorbitanmonolaurate, triethanolamine sodium acetate, triethanolamineoleate, sodiumlauryl sulfate, dioctyl sodium sulfosuccinate, polyoxyethylenes orbit and fatty acid esters, etc.

Suitable glidants include magnesium trisilicate, powdered cellulose, starch, talc, tribasic calcium phosphate, stearate salts and colloidal silicon dioxide. Most preferred glidants are talc, magnesium stearate and colloidal silicon dioxide.

The present invention is further illustrated by the following examples which are provided merely to be exemplary of the invention and do not limit the scope of the invention. Certain modifications and equivalents will be apparent to those skilled in the art and are intended to be included within the scope of the present invention.

## EXAMPLES

## Example 1: Composition and preparation of Chlorophyllin Tablets

a) Table 1: Formula Composition of Chlorophyllin Dispersible Tablets

| Sr.No | Ingredients | Range (%w/w) |
|-------|-------------|--------------|
| 1 | Sodium copper Chlorophyllin | 30 -70 |

(continued)

| Sr.No | Ingredients | Range (%w/w) |
|---|---|---|
| 2 | Mannitol | 20 -50 |
| 3 | Lactose monohydrate | 1-20 |
| 4 | Croscarmellose sodium | 1-15 |
| 5 | Sucralose | 0.5 -5 |
| 6 | Colloidal silicon dioxide | 0.25 -3 |
| 7 | Magnesium stearate | 0.25 -3 |
| 8 | Total | 100.00 |

**Procedure:**

Chlorophyllin is combined along with all excipients except magnesium stearate and sieved through a suitable mesh screen and material is transferred into an octagonal blender where it is blended for sufficient time. Magnesium stearate is then passed through a suitable mesh screen and added to the blended material in the blender. The contents are then lubricated for sufficient time and discharged for compression. Using a rotary tablet press fitted with suitable punches and dies of the desired shape and size, the lubricated blend is compressed into tablets.

**b) Table 2: Formula Composition of Chlorophyllin Effervescent Tablets**

| Sr.No | Ingredients | Range (%w/w) |
|---|---|---|
| 1 | Sodium Copper Chlorophyllin | 20-60 |
| 2 | Mannitol # | 5 -50 |
| 3 | Citric acid (Anhydrous) | 0.5-10 |
| 4 | Tartaric acid | 5-15 |
| 5 | Sodium bicarbonate | 5-25 |
| 6 | Croscarmellose sodium | 0.25-6 |
| 7 | Sucralose | 0.25-5 |
| 8 | Colloidal silicon dioxide | 0.25-5 |
| 9 | Sodium benzoate | 0.25-5 |
| 10 | Total | 100.00 |

**Procedure:**

Chlorophyllin is combined along with all excipients except magnesium stearate and sieved through a suitable mesh screen and material is transferred into an blender where it is blended, compressed the blend using a rotary tablet press fitted with suitable punches and dies of the desired shape and size.

**c) Table 3: Formula composition of Chlorophyllin oral tablets**

| Sr. No. | Name of Ingredients | Range (%w/w) |
|---|---|---|
| 1 | Sodium Copper Chlorophyllin | 20-80 |
| 2 | Microcrystalline Cellulose | 15-50 |
| 3 | Lactose Monohydrate | 1-15 |
| 4 | Croscarmellose Sodium | 0.25-10 |
| 5 | Povidone | 0.25-5 |
| 6 | Colloidal Silicon dioxide | 0.25-5 |

(continued)

| Sr. No. | Name of Ingredients | Range (%w/w) |
|---------|---------------------|--------------|
| 7 | Magnesium Stearate | 0.25-5 |
| 8 | Coating material | 0.25-5 |
| **Total** | | 100.00 |

**Procedure:**

Chlorophyllin is combined along with intragranular excipients into a high shear granulator and initially dry mixed. While mixing in RMG, binder solution is added to the mixing powders to granulate the materials continues until the desired granulation end point is achieved. The resulting granules added to a fluid bed drier and dried at 60°C until the desired L.O.D (loss on drying) is achieved. The dried material is sieved through a suitable mesh screen. The dried and sized material is combined with pre sieved extra granular material into blender, mixed. Magnesium stearate is then passed through a suitable mesh and added to the blended material in the blender. The contents are then lubricated for sufficient minutes and discharged for compression. Using a rotary tablet press fitted with suitable punches and dies of the desired shape and size, the lubricated blend is compressed into tablets. The tablet cores are then placed into a side vented, fully perforated coating pan where they are coated with 7% solids content of Opagloss 2 in water until a theoretical weight gain of 2% is achieved.

Chlorophyllin powder used for manufacture of the tablets in the present invention contains ~ 40% copper isochlorin e4.]

**Example 2: Determination of the selective level dose of Chlorophyllin.**

In vitro studies demonstrate that Chlorophyllin inhibits SARS-CoV-2 multiplication. It was found that the concentration of CHL required to inhibit the growth of SARS-CoV-2 virus by 50% (EC50) was less than 1 micro molar. At 5 micro-molar concentration, Chlorophyllin inhibited SARS-CoV-2 multiplication by about 87%. In this experiment, the time required for inhibition of virus by chlorophyllin was 72 hr. Therefore, it is implied that serum levels of chlorophyllin need to be maintained at higher than EC50 concentration for more than 3 days.

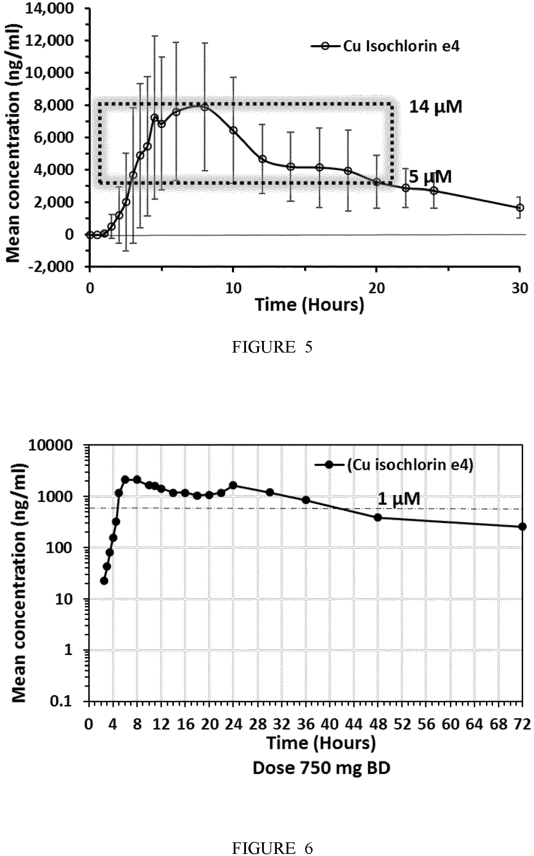

Based on Fig 4(B), it was calculated that a serum concentration of more than 5 micro molar copper-isochlorin e4 needs to be maintained for 3 days (72 hr) to achieve 87% inhibition of SARS-CoV-2 infection. To achieve this concentration of Copper isochlorin e4, 750 mg tablet of chlorophyllin tablet was effective (Figure 5).

[One micro molar in vitro concentration of chlorophyllin in Fig 4B is equivalent to 0.4 micro molar Copper isochlorin e4 concentration in Fig 5].

Basis for determining the Dose of Chlorophyllin tablet of 750 mg:

Molecular weight of Copper isochlorin e4 is 615. The maximum serum concentration (Cmax) of Copper isochlorin in human serum was 8637 ng/ml. = 8637 micrograms / liter.

$$8637 \text{ micrograms / liter divided by molecular weight } 615 = 14.00813 \text{ micro Moles per liter}$$

Since 750mg chlorophyllin tablet achieved a serum concentration of Copper isochlorin e4 at 7 micro molar to 14 micro molar concentration for almost two third of the day (18 hr), it was calculated that 750 mg daily dose will achieve the desired levels of the active ingredient in humans and the levels will be maintained above EC50 concentration for 24 hr. Fig 4 also shows that higher concentration of chlorophyllin may be more beneficial. Hence, the wider range of chlorophyllin for medicament of COVID-19 disease is proposed to be 500 to 1500 mg tablet per day.

The chlorophyllin tablets are given for three to fourteen consecutive days before or after appearance of symptoms in COVID-19 patients. If the prescribed regime is not followed, chlorophyllin tablets are less effective.

At 5$\mu$M Concentration CHL inhibited growth of SARS-CoV-2 by ~87% without causing cytotoxicity in the target cells (Fig 4B right panel). This concentration can be achieved in human serum using the 750 mg oral tablets manufactured by IDRS Labs Pvt Ltd (Fig 5).

Cmax in human serum after administration of oral tablet of 750 mg chlorophyllin was found to be 8637.7 ng/ml which corresponds to about 14 micro molar concentration (Fig 5). This concentration is at least 14 times higher than the EC50 concentration of chlorophyllin required to inhibit SARS-CoV-2 multiplication.

**EXAMPLE 3:**

**i. Safety, tolerability and toxico-kinetics:**

Different doses of Chlorophyllin (100 to 5000 mg/kg body weight) were given to mice and rats (6 males, 6 females) by oral gavage in an accredited facility for animal toxicity testing. It was found that an acute dose of up to 5000 mg/kg body weight was well tolerated in rodents. In a repeat dose toxicity study, a daily dose of 1000 mg per kg body weight Chlorophyllin was given for 28 consecutive days and it was found to be well tolerated. There were no signs of toxicity in rodents treated with indicated doses of Chlorophyllin.

**ii. Human subjects & dosing:**

An open label Phase I clinical study was conducted to assess pharmacodynamics of sodium copper Chlorophyllin in healthy adult, human male subjects under fasting conditions. The study was approved by Drug Controller General of India (DCGI). The trial is registered with the Clinical Trial Registry of India (CTRI/2018/09/015576). Five healthy male subjects were allowed to fast overnight and one Chlorophyllin tablet (750 mg) was administered to each subject by oral route along with ~200 ml water. The subjects were given breakfast, lunch and snacks and a second tablet of Chlorophyllin (750 mg) was administered to each subject by oral route along with 200 ml water after 12 hr.

RESULT: Chlorophyllin was well tolerated at 5000 mg/kg body weight acute oral dose in mice and rats. Further, it was well tolerated at 1000 mg/kg body weight dose in a 28 day repeat dose study in rats. Since it did not induce any sign of toxicity, morbidity or mortality in rodents at these doses, LD50 dose of Chlorophyllin is proposed to be much higher than 5000 mg/kg body weight in rodents.

Experimental results indicate that the concentration of chlorophyllin required for ~90% inhibition of multiplication of SARS-CoV-2 virus is about 5 micro molar (Fig 4). In order to achieve this concentration, a dose of chlorophyllin in excess of 500 mg per day is required. The preferred dose is 750 mg per day and broad steps of dose regime for treatment of COVID-19 would involve administration of a high dose of chlorophyllin (500 mg to 1500mg per day). The duration of treatment can vary from 3 days to 14 days depending upon the severity of the infection and condition of the patient.

Since less than 1 micro-molar concentration of chlorophyllin did not significantly decrease the multiplication of SARS-CoV-2 virus, a lower dose (less than 500 mg/day) is not anticipated to be effective in treatment of COVID-19 disease (Fig 4 right panel, 0.1 micro molar concentration of chlorophyllin did not inhibit SARS-CoV-2 virus multiplication). For all other indications including internal deodorant, cancer chemo-prevention and wound healing, the dose of chlorophyllin used in humans is between 100 to 300 mg per day which is lower than the proposed dose regimen.

If the prescribed regime is not followed, chlorophyllin tablets will be less effective. A specific example is give below.

750 mg tablet of chlorophyllin was given to healthy human subjects after breakfast. In this case the overnight fasting regime was changed. The change in regime led to lesser serum concentration of chlorophyllin (between 1 to 2 micro molar) as shown in the figure 6.

**ii) Assessment of pharmacodynamics:**

a) The serum concentration of Copper isochlorin e4 was measured by LC-MS/MS. The formulation development and design of tablets was aimed to improve bioavailability of chlorophyllin by oral route. Surprisingly it was noted that Copper isochlorin e4 level increased on administering the present formulation in the specific dose regime to high enough levels in human serum which can inhibit SARS-CoV-2 virus.

The observed increase in the serum levels of Sodium Copper isochlorin e4 is a net result of formulation (binders etc) and dose of the tablets. The role of binding/carrier molecules of the formulation is aimed at increasing the uptake of copper isochlorin e4 in human blood. Since there is no prior-art on increase in the serum levels of Sodium Copper isochlorin e4 using existing tablets of chlorophyllin (100mg x 3 tablets per day), it implies that increasing the dose of existing chlorophyllin tablets available in the market will not increase the serum concentration of Sodium Copper isochlorin e4 to desired levels.

Surprisingly and unexpectedly experimental Data showed that higher level of administration (1500 mg BD) causes an increase in the serum concentration of Copper isochlorin e4 in human serum which is almost 2 times higher than the concentration achieved with 750 mg.

Based on this data a dose dependent increase in Copper isochlorin e4 in human serum after administration of 750 mg and 1500 mg tablets is being observed. Hence, using back extrapolation of the curve, it can be interpreted that lower dose of chlorophyllin tablets from the present invention will also not be able to achieve and sustain the desired levels of active pharmaceutical ingredient in human serum for medicament of COVID-19 disease.

b) Assessment of abundance of hematopoietic stem cells, absolute neutrophil count (ANC) and white blood cell (WBC) counts and concentration of chemokines in human blood were done.

Chlorophyllin tablets comprising Chlorophyllin or salts thereof (750 mg), were administered to healthy human subjects after overnight fasting. Blood samples were collected through an indwelling intravenous cannula (Venflon) placed in the fore arm vein of the subjects at different time intervals. Absolute neutrophil count (ANC) and white blood cell (WBC) counts were measured. Serum was separated and chemokine concentration in the serum was measured using cytometric bead array.

Blood sample at each time point was also collected in K2EDTA vacutainer tube and erythrocytes were lysed using RBC lysis buffer. Then cells were stained with fixable far red live dead reagent and PE-CD34 and FITC-CD45 antibodies at 4°C and fixed with 4% formaldehyde. The frequency of CD34+ hematopoietic stem and progenitor cells in the blood was determined by flow cytometry and FlowJo software.

RESULTS: Chlorophyllin was well tolerated at 750 mg given twice a day in all five human subjects. An increase in absolute neutrophil count (ANC) relative to pre-dose value was seen in all five subjects (Fig 1A, 1B). The increase in ANC was between 12.4-32.5% relative to pre-dose value at 8-16 hr after first dose of Chlorophyllin. ANC increase was observed within 8hr after first dose of Chlorophyllin (750 mg), and 4hr before second dose. Chlorophyllin administration also increased white blood cell count in the blood. The increase in WBC count was between 12.8-26.5% relative to pre-dose value at 8-16hr after first dose of Chlorophyllin (Fig 1C, 1D).

The abundance of CD34+ hematopoietic stem and progenitor cells in the blood was significantly decreased as compared to pre-dose value in all five human subjects. The relative decrease in percentage of CD34+ HPSCs in human blood was between 50-85% as compared to pre-dose value (Fig 2).

Chlorophyllin administration significantly decreased the concentration of pro-inflammatory chemokines MCP-1, MIP-1α and MIP-1β. The relative decrease in MCP-1 in human blood was between 13-37% as compared to pre-dose value 24 hr after first dose of Chlorophyllin (Fig 3).

The relative decrease in MIP-1α in human blood was between 12-21% as compared to pre-dose value 72hr after first dose of Chlorophyllin(Fig 3).

The relative decrease in MIP-1β in human blood was between 11-55% as compared to pre-dose value 24hr after first dose of Chlorophyllin (Fig 3).

Chlorophyllin administration resulted in up to 30% increase in the lymphocyte counts in human blood as compared to pre-dose value (Fig 4A).

Administration of Chlorophyllin tablets reached serum concentration of copper isochlorin e4 in human blood to 5-35 μM.

### iii) Assessment of anti-viral activity:

Vero CCL 81 cells were infected with SARS-CoV-2 virus and treated with different concentrations (0,0.1,1.0.5.0,25.0 or 50μM) of Chlorophyllin for 72 hr. Virus control sample was not treated with the drug. RNA was isolated from each sample after 72 hr and was subjected to quantitative-real time polymerase chain reaction for the viral RdRp-2 gene. The gene copy number in each sample was used for quantification of viral titer in each sample.

RESULTS: Chlorophyllin inhibited viral multiplication in Vero CCL-81 cells in a dose dependent manner in vitro. The concentration of Chlorophyllin required for inhibition of 50% viral multiplication(EC50) was less than 1 μM and 5 micro-molar concentrations of Chlorophyllin inhibited the growth of SARS-CoV-2 virus by ~87% in three days' time in vitro. Administration of Chlorophyllin tablets reached serum concentration of Copper isochlorin e4 in human blood to 5-35 μM, which is higher than the IC50 concentration for inhibition of SARS-CoV-2 multiplication (Fig 4B).

The mean serum concentration of Copper isochlorin e4 in human blood was higher than 5 micro-molar (Fig 5) after administration of oral tablets of CHL of the present invention. At 5 micro-molar concentration, Chlorophyllin inhibited the multiplication of SARS-CoV-2 virus by more than 87% (Fig 4B). The inhibition of viral multiplication and improved lymphocyte count (Fig 4A) demonstrate the therapeutic effects of Chlorophyllin in the form of oral composition.

Present advancement provides a pharmaceutical composition comprising synergistic combination of chlorophyllin or pharmaceutically acceptable salts thereof along with carrier/binding material with demonstrated safety, tolerability and pharmacodynamics in human. This composition when administered in oral tablet from at a specific dose/regime achieve enhanced serum concentration of Copper isochlorin-e4 in human blood up to a concentration of 5 to 35 μM which can be used for effective prevention, mitigation and treatment of corona virus infections including COVID-19. It may provide a novel medicament for COVID-19 via suppression of SARS-CoV-2 virus multiplication, faster recovery from lymphocytopenia, increased production of neutrophils, suppression of inflammation and prevention of oxidative-stress in lung epithelial cells. The dose of Chlorophyllin (750 mg) once a day for three to fourteen days is based on an adult human weighing 50 Kg to 100 Kg and it may be modified depending upon disease severity, weight, age, sex and condition of the patient. The said composition is also responsible for improving other hematological parameters in human and live-stock animals.

**EXAMPLE 4: To illustrate that chlorophyllin dose regime is effective under fasting state but not after food**

**1. CD34+ counts:**

Comparative evaluation for each time point with respect to baseline for CD34+ counts is summarized as below. Chlorophyllin oral tablet decreased CD34+ HSPC in blood under fasting conditions but not after food.

|  | Mean (+/- SD) p-value | |
| --- | --- | --- |
|  | After overnight fasting | After breakfast |
| T0-T24 | 0.0804 (0.0374) 0.0086 | 0.0113 (0.0101) 0.1918 |
| TO-T96 | 0.0494 (0.0243) 0.0105 | 0.00567 (0.0196) 0.6655 |

**2. Absolute Neutrophil Count:**

Comparative evaluation for each time point with respect to baseline for ANC is summarized as below. Chlorophyllin oral tablet increased neutrophil counts in blood under fasting conditions but not after food.

|  | Mean (+/- SD) p-value | |
| --- | --- | --- |
|  | After overnight fasting | After breakfast |
| T0-T8 | -0.6520 (0.3140) 0.0097 | -0.6500 (0.3487) 0.0840 |

**3. Macrophage Inflammatory Protein 1 beta:**

Descriptive statistics of each time point for Macrophage Inflammatory Protein 1 beta is summarized as below. Chlorophyllin oral tablet decreased MIP 1 beta in blood under fasting conditions but not after food.

|  | Mean (+/- SD) p-value | |
| --- | --- | --- |
|  | After overnight fasting | After breakfast |
| 0.000 hr (pre dose) | 42.668 (12.3837) | 21.427 (0.9256) |
| 16.000 hr | 26.026 (9.1841) | 29.380 (4.4145) |
| 24.000 hr | 32.274 (14.3207) | 19.018 |

The above three examples provide comparison of effect of chlorophyllin tablets under fasting and fed state (after breakfast). The serum levels of copper isochlorin e4 are higher under fasting state as compared to after breakfast (Fig 5 vs. Fig 6).

The efficacy of chlorophyllin in reducing CD34+ HSPCs and MIP1 beta concentration and increasing absolute neutrophil count in human blood was also reduced when the said dose regime was changed from fasting to fed state (after breakfast).

Hence the present invention provides the pharmaceutical composition comprising of Chlorophyllin or salts thereof and its utilities including method of treatment, enabling the absorption of Copper-isochlorin e4 into human blood that results in an increase in lymphocyte count and decrease in the abundance of hematopoietic stem and progenitor cells (HPSCs) in human blood. The said composition is useful for the treatment of corona virus disease caused by SARS-CoV-2 infection by decreasing viral infection, reducing cytokine storm and pro-inflammatory chemokines, reducing epithelial-cell oxidative-stress in the lungs and increasing the production of leukocytes.

**Claims**

1.  A chlorophyllin containing pharmaceutical composition for prevention of pathogenesis of coronavirus disease in humans comprising:

    Chlorophyllin or salts thereof in amounts between 20 to 80% by wt;
    and a pharmaceutically acceptable adjuvant.

2.  The chlorophyllin containing pharmaceutical composition as claimed in claim 1 comprising water-soluble derivative of green plant pigment chlorophyll wherein the composition is either a solid oral dosage form or liquid oral dosage form suitable for oral administration

3.  The chlorophyllin containing pharmaceutical composition according to the claim 2, wherein the composition is a tablet dosage form, preferably a coated tablet.

4.  The chlorophyllin containing pharmaceutical composition according to the claim 1, which achieves serum concentration of an active pharmacological ingredient Copper-isochlorin-e4 up to a concentration range of 5 to 35 micromolar in human blood to inhibit SARS-CoV-2 infection.

5.  The chlorophyllin containing pharmaceutical composition according to claim 1, which induces 10 to 25% increase in blood neutrophil counts, decrease in pro-inflammatory chemokines (1) chemokine ligand 2 or Monocyte Chemoattractant Protein-1 (MCP-1); (2) Chemokine (C-C motif) ligand-3 or Macrophage Inflammatory Protein (MIP-1-alpha) and (3) Chemokine (C-C motif) ligand 4 or Macrophage Inflammatory Protein (MIP-1-beta) which are associated with pathogenesis of COVID-19 in human.

6.  The chlorophyllin containing pharmaceutical composition according to claim 1 favoring selectively anyone or more of:

    i) increase in absolute neutrophil count (ANC) between 12.4-32.5% relative to pre-dose value at 8-16 hr after first dose of Chlorophyllin(750 mg) (Fig. 1A, 1B);
    ii) increase in WBC count between 12.8-26.5% relative to pre-dose value at 8-16 hr after first dose of Chlorophyllin(Fig 1C ,1D).
    iii) decreasing the abundance of CD34+ hematopoietic stem and progenitor cells in the blood between 50-85% as compared to pre-dose value (Fig 2) in human subjects.
    iv) decreasing the concentration of pro-inflammatory chemokines MCP-1, MIP-1-alpha and MIP-1-beta between 13-37% as compared to pre-dose value 24 hr after first dose of Chlorophyllin (Fig 3).
    v) relative decrease in MIP-1-alpha in human blood between 12-21% as compared to pre-dose value 72 hr after first dose of Chlorophyllin.
    vi) relative decrease in MIP-1-beta in human blood between 11-55% as compared to pre-dose value 24 hr after first dose of Chlorophyllin.
    vii) inducing up to 30% increase in human blood lymphocyte counts.

7.  A method of treatment of disease condition including pathogenesis of coronavirus disease in humans comprising administering preferably via oral administration of pharmaceutically effective amount of Chlorophyllin or salts thereof at a dose of 500 to 1500 mg, preferably 750 mg for 3 to 14 days to human adult weighing 50-100 Kg to thereby induce an increase in the serum concentration of an active pharmacological ingredient (API) Copper isochlorin e4 up to a concentration range of 5 to 35 microM in human blood to inhibit SARS-CoV-2 infection.

8.  The method as claimed in claim 7 comprising of administration of one tablet containing at least 500 mg preferably about 750 mg Chlorophyllin every day in the morning for 3 to 14 consecutive days with a dose regimen wherein the tablet must be taken 10 to 12 hr after overnight fasting along with water and food should be consumed 2 hr after administration of the Chlorophyllin tablet.

9.  The method as claimed in claim 7, comprising administering said dosage of Chlorophyllin further provides for reducing the abundance of CD34 + hematopoietic stem and progenitor cells (HSPCs) in human blood.

10. The method as claimed in claim 7 comprising administering said dosage further providing for inducing up to 30% increase in human blood lymphocyte counts.

**11.** The method as claimed in of claim 7 comprising administering said dosage further for inducing 10 to 25% increase in blood neutrophil counts, decrease in pro-inflammatory chemokines (1) chemokine ligand 2 or Monocyte Chemoattractant Protein-1 (MCP-1); (2) Chemokine (C-C motif) ligand-3 or Macrophage Inflammatory Protein MIP-1-alpha and (3) Chemokine (C-C motif) ligand 4 or Macrophage Inflammatory Protein MIP-1-beta) which are associated with pathogenesis of COVID-19 in human.

**12.** The method as claimed in claim 7 comprising administering said dosage thereby favoring selectively anyone or more of:

> i) increase in absolute neutrophil count (ANC) between 12.4-32.5% relative to pre-dose value at 8-16 hr after first dose of Chlorophyllin (750 mg) (Fig 1A, 1B);
> ii) increase in WBC count between 12.8-26.5% relative to pre-dose value at 8-16hr after first dose of Chlorophyllin (Fig 1C , ID);
> iii) decreasing the abundance of CD34+ hematopoietic stem and progenitor cells in the blood between 50-85% as compared to pre-dose value (Fig 2) in human subjects.
> iv) decreasing the concentration of pro-inflammatory chemokines MCP-1, MIP-1-alpha and MIP-1-beta. between 13-37% as compared to pre-dose value 24 hr after first dose of Chlorophyllin (Fig 3);
> v) relative decrease in MIP-1-alpha in human blood between 12-21% as compared to pre-dose value 72 hr after first dose of Chlorophyllin;
> vi) relative decrease in MIP-1-beta in human blood was between 11-55% as compared to pre-dose value 24 hr after first dose of Chlorophyllin.

**13.** Chlorophyllin or salts thereof in amounts between 20 to 80% by wt. and pharmaceutically acceptable adjuvant in dosage level of 500 to 1500 mg, for use in treatment of pathogenesis of coronavirus disease in humans by inducing an increase in the serum concentration of an active pharmacological ingredient (API) Copper isochlorin e4 up to a concentration range of 5 to 35 micro M in human blood to inhibit SARS-CoV-2 infection.

**14.** The Chlorophyllin or salts thereof as claimed in claim 13 for use either as a solid oral dosage form or liquid oral dosage form suitable for oral administration

**15.** The chlorophyllin or salt thereof as claimed in claim 13 for inducing 10 to 25% increase in blood neutrophil counts, decrease in pro-inflammatory chemokines (1) chemokine ligand 2 or Monocyte Chemoattractant Protein-1 (MCP-1); (2) Chemokine (C-C motif) ligand-3 or Macrophage Inflammatory Protein (MIP-1-alpha) and (3) Chemokine (C-C motif) ligand 4 or Macrophage Inflammatory Protein (MIP-1-beta) which are associated with pathogenesis of COVID-19 in human.

**16.** The chlorophyllin or salt thereof as claimed in claim 12 for selectively anyone or more of:

> i) increase in absolute neutrophil count (ANC) between 12.4-32.5% relative to pre-dose value at 8-16 hr after first dose of Chlorophyllin (750 mg) (Fig 1A, 1B);
> ii) increase in WBC count between 12.8-26.5% relative to pre-dose value at 8-16 hr after first dose of Chlorophyllin (Fig 1C ,1D).
> iii) decreasing the abundance of CD34+ hematopoietic stem and progenitor cells in the blood between 50-85% as compared to pre-dose value (Fig 2) in human subjects.
> iv) decreasing the concentration of pro-inflammatory chemokines MCP-1, MIP-1-alpha and MIP-1-beta between 13-37% as compared to pre-dose value 24 hr after first dose of Chlorophyllin (Fig 3).
> v) relative decrease in MIP-1-alpha in human blood between 12-21% as compared to pre-dose value 72 hr after first dose of Chlorophyllin.
> vi) relative decrease in MIP-1-beta in human blood was between 11-55% as compared to pre-dose value 24 hr after first dose of Chlorophyllin.

**17.** Chlorophyllin or salt thereof for use in manufacture of medicament in amounts between 20 to 80% by wt. and pharmaceutically acceptable adjuvant in dosage level of 500 to 1500 mg, for treatment of pathogenesis of coronavirus disease in humans by inducing an increase in the serum concentration of an active pharmacological ingredient (API) Copper isochlorin e4 up to a concentration range of 5 to 35 microM in human blood to inhibit SARS-CoV-2 infection.

**18.** The chlorophyllin containing pharmaceutical composition according to the claim 1 which increases bioavailability of the Chlorophyllin in human subjects.

FIGURE 1

FIGURE 2

FIGURE 3

A                                                                                          B

FIGURE 4

FIGURE 5

FIGURE 6

Europäisches
Patentamt

European
Patent Office

Office européen
des brevets

## EUROPEAN SEARCH REPORT

Application Number

EP 21 16 2765

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/325915 A1 (SAVANGIKAR CHITRA VASANT [IN] ET AL) 15 November 2018 (2018-11-15) * Example 2, composition 4; example 2 * | 1,2,4-6, 8-18 | INV. A61K9/20 A61K31/555 A61P11/00 A61P31/14 |
| Y | US 2012/082720 A1 (ANG SAM POON [US]) 5 April 2012 (2012-04-05) * paragraph [0069]; claim 1 * | 1-3 | |
| Y | CN 111 329 859 A (UNIV FUDAN) 26 June 2020 (2020-06-26) * claims 1-6; example 1 * | 1-3 | |
| A | US 2017/290843 A1 (SHARMA DEEPAK [IN] ET AL) 12 October 2017 (2017-10-12) * claims 1-16 * | 1-18 | |
| A | US 2007/148222 A1 (DOROGI PETER L [US] ET AL) 28 June 2007 (2007-06-28) * claims 1-20 * | 1-18 | |

TECHNICAL FIELDS
SEARCHED      (IPC)

A61K
A61P

The present search report has been drawn up for all claims

| Place of search | Date of completion of the search | Examiner |
|---|---|---|
| Munich | 17 August 2021 | Wörth, Christian |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 21 16 2765

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2021

| Patent document cited in search report | | Publication date | Patent family member(s) | | Publication date |
|---|---|---|---|---|---|
| US 2018325915 | A1 | 15-11-2018 | AU 2016210540 A1 | | 10-08-2017 |
| | | | BR 112017015338 A2 | | 09-01-2018 |
| | | | CN 107427417 A | | 01-12-2017 |
| | | | EA 201791319 A1 | | 31-01-2018 |
| | | | EP 3247323 A2 | | 29-11-2017 |
| | | | JP 6772151 B2 | | 21-10-2020 |
| | | | JP 2018502893 A | | 01-02-2018 |
| | | | JP 2021006555 A | | 21-01-2021 |
| | | | SG 11201705694V A | | 30-08-2017 |
| | | | US 2018325915 A1 | | 15-11-2018 |
| | | | WO 2016116950 A2 | | 28-07-2016 |
| US 2012082720 | A1 | 05-04-2012 | NONE | | |
| CN 111329859 | A | 26-06-2020 | NONE | | |
| US 2017290843 | A1 | 12-10-2017 | NONE | | |
| US 2007148222 | A1 | 28-06-2007 | US 2007148222 A1 | | 28-06-2007 |
| | | | US 2007148223 A1 | | 28-06-2007 |
| | | | US 2010247591 A1 | | 30-09-2010 |
| | | | US 2010247630 A1 | | 30-09-2010 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Chem. Res. Toxicol.,* 2000, vol. 13 (9), 900-906